# EUROPEAN PATENT APPLICATION

(11) **EP 3 734 550 A1**
(43) Date of publication of application: **04.11.2020**
(21) Application number: 19172166.1
(22) Date of filing: 02.05.2019
(51) Int. Cl.: G06T 7/11

(54) **REMOVAL OF FALSE POSITIVES FROM WHITE MATTER FIBER TRACTS**

(71) Applicant: Koninklijke Philips N.V., 5656 AG Eindhoven (NL)
(72) Inventor: SCHWAB, Evan, 5656 AE Eindhoven (NL); EWALD, Arne, 5656 AE Eindhoven (NL)
(74) Representative: de Haan, Poul Erik

(57) **Abstract**

The invention provides for a medical imaging system (100, 400). comprising:
The execution of the machine executable instructions (112) causes a processor (104) to: receive (200) a set of input white matter fiber tracts (118); receive (202) the label from a discriminator neural network (116) in response to inputting the set of input white matter fiber tracts; generate (204) an optimized feature vector (122) using the set of input white matter fiber tracts and a generator neural network ((114) if the label indicates anatomically incorrect; receive (206) the set of generated white matter fiber tracts from the generator neural network in response to inputting the optimized feature vector; and construct (208) a false positive subset (126) of the set of input white matter fiber tracts using the generated set of white matter fiber tracts.

## Description

### FIELD OF THE INVENTION

The invention relates to magnetic resonance imaging, in particular to diffusion tensor imaging.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B₀ field or the main magnetic field. Gradient magnetic fields and magnetic fields (the B₁ field) caused by radio frequency (RF) pulses may be used to manipulate the orientation of spins. If the B₁ field rotates spins out of alignment with the B0 field they will begin to process and generate a radio frequency signal.

In Diffusion Weighted Imaging (DWI) after spins have been rotated to an angle with respect to the B₀ field a first diffusion weighted gradient is used to de-phase spins within a volume. A second identical diffusion weighted gradient then rephases the spins within the volume. After the spins are rephased, their radio frequency signal can be measured. If spins move in or out of the volume, they will not be correctly rephased. DWI is directionally sensitive. If DWI is preformed in multiple directions, a diffusion tensor image can be calculated. A diffusion tensor image is useful for example in determining the location of white matter fiber tracts in the brain. Diffusion tensor imaging is for example reviewed in Bihan et. al., "Diffusion Tensor Imaging: Concepts and Applications," Journal of Magnetic Resonance Imaging 13:534-5436 (2001).

Chinese patent application publication CN 106971410 A discloses a white matter fiber tract reconstruction method based on deep learning, which mainly aims at white matter fiber tract reconstruction. The method comprises the following steps: extracting signal sparsity features of a digital image in a training sample set; importing the signal sparsity features to a convolutional neural network for training, forwardly propagating the classification result, using a back propagation algorithm for the classification error, and getting a best network model; inputting extracted signal sparsity features of an image in a test sample set to a trained network model to get a final prediction result; and finally, describing the result into the orientation and distribution of white matter fibers through continuous curve fitting, and reconstructing a three-dimensional white matter fiber tract. Cross and forked white matter fiber tracts can be constructed accurately to provide help for the clinical research and physiological and pathological mechanisms of white matter fiber tracts.

### SUMMARY OF THE INVENTION

The invention provides for a medical imaging system, a computer program product, and a method in the independent claims. Embodiments are given in the dependent claims.

When mapping white matter fiber tracts using diffusion tensor imaging a common problem is that false positive white matter fiber tracts are produced. The false positive white matter fiber tracts are tracts which are not actually in the brain but are an artifact of the algorithm which uses the diffusion tensor image to determine the input white matter fiber tracts. Embodiments may provide for a means of removing the false positive fiber tracts. As a result, embodiments may be considered to be an enhancement to practically any existing diffusion tensor tractography algorithm, manual or automatic.

To do this a generative adversarial network (GAN) is trained or constructed. The GAN comprise a generator neural network and a discriminator neural network. The generator neural network is trained to take a feature vector as an input and output a set of generated white matter tracts. A single white matter tract is a collection of connected points. The discriminator is trained to identify a set of input white matter tracts as being anatomically correct or anatomically incorrect. The generator neural network and the discriminator neural network are trained together.

The set of input white matter tracts are input into the discriminator neural network and the entire set is labeled as either being anatomically correct or anatomically incorrect. If they are labeled as being anatomically incorrect, then the generator neural network is used to generate a set of generated white matter fiber tracts which match the set of input white matter tracts as closely as possible. This is done by taking the set of input white matter tracts and working backwards with the generator neural network to generate an optimized feature vector. This for example can be accomplished using backpropagation. The optimized feature vector is then input into the generator neural network to produce the set of generated white matter fiber tracts. By comparing the set of input white matter tracts to the set of generated white matter fiber tracts, tracts which are likely false positive white matter tracts can be identified and added to a false positive subset of the set of input white matter fiber tracts.

A medical imaging system as used herein encompasses a workstation or data processing system that is able to perform calculations for image processing, data manipulation, and/or the reconstruction of medical imaging data in two medical images or into processed medical image data. Medical image data is data which may be rendered into an image. A medical imaging system may also encompass the apparatus for acquiring medical imaging data such as magnetic resonance imaging data. For example, the medical imaging system may also incorporate a magnetic resonance imaging system and/or other types of medical scanners.

In one respect the invention provides for a medical imaging system that comprises a memory for storing machine-executable instructions and a generative adversarial neural network. This is also typically known as a GAN. The generative adversarial neural network comprises a generator neural network and a discriminator neural network. The generator neural network is configured for outputting a set of generated white matter fiber tracts in response to inputting a feature vector. The discriminator neural network is configured for outputting a label in response to inputting a set of input white matter fiber tracts. The label indicates the set of input white matter fiber tracts as anatomically correct or anatomically incorrect. In diffusor tensor imaging for magnetic resonance imaging the diffusion tensor of brain tissue may be used to track the position and direction of white matter fiber tracts. In reference to the invention the generated white matter fiber tracts and the input white matter fiber tracts are represented as a set of connected points. They represent the path of an individual white matter fiber tract.

The medical imaging system further comprises a processor for controlling the medical imaging system. Execution of the machine-executable instructions further cause the processor to receive the set of input white matter fiber tracts. The set of input white matter fiber tracts may be received via a network connection or retrieved from a memory. They may also be derived or calculated from a diffusion tensor. Execution of the machine-executable instructions further cause the processor to receive the label from the discriminator neural network in response to inputting the set of input white matter fiber tracts. The label is for the set of input white matter fiber tracts as a whole. The discriminator neural network is able to discriminate if the set of input white matter fiber tracts appear to be anatomically correct or anatomically incorrect.

Execution of the machine-executable instructions further causes the processor to generate an optimized feature vector using the set of input white matter fiber tracts and the generator neural network if the label indicates anatomically incorrect. If the label indicates anatomically correct then for example the set of input white matter fiber tracts may be accepted as being correct. The optimized feature vector may for example be generated by using the techniques of back propagation. Execution of the machine-executable instructions further cause the processor to receive the set of generated white matter fiber tracts from the generator neural network in response to inputting the optimized feature vector.

For example, the optimized feature vector maybe used to generate the closest set of generated white matter fiber tracts to the set of input white matter fiber tracts. The closest set of generated white matter fiber tracts represents the closest set of anatomically correct white matter fiber tracts within the context of the training space of the generator neural network. Doing this enables a comparison between the two sets of white matter fiber tracts. Execution of the machine-executable instructions then further causes the processor to construct a false positive subset of the set of input white matter fiber tracts using the generated set of white matter fiber tracts as a reference. This step may for example be performed by making a comparison between the set of generated white matter fiber tracts and the input white matter fiber tracts. Individual white matter fiber tracts that do not match the fiber tracts of the set of generated white matter fiber tracts may for example be assigned to the false positive subset.

This embodiment may be beneficial because when determining the path and location of white matter fiber tracts from diffusion tensor imaging there is a tendency to produce false positive white matter fiber tracts. This embodiment may provide an efficient means of automatically identifying these false positive white matter fiber tracts.

In another embodiment execution of the machine-executable instructions further causes the processor to render the set of white matter fiber tracts on a display if the label indicates anatomically correct. This may also cause the processor to store the white matter fiber tracts for further use or with other image data such as a DICOM image.

In another embodiment execution of the machine-executable instructions further cause the processor to render the set of input fiber tracts on the display. Execution of the machine-executable instructions further causes the processor to indicate the false positive subset on the display and/or indicate an anatomically correct subset on the display using the false positive subset. In this embodiment the entire group of the set of input fiber tracts are rendered on a display and the false positive subset is used in one way or the other to make clear to the user which tracts are false positive ones and which ones are identified as being anatomically correct. This for example may be useful when a physician or the healthcare professional is inspecting the data.

In another embodiment execution of the machine-executable instructions provides a user interface which may be configured to allow removal of the false positive subset. This for example may be provided by an individual control which enables the entire false positive subset to be removed from the set of input white matter fiber tracts or which may be used to remove a portion of the false positive subset.

In another embodiment execution of the machine-executable instructions further causes the processor to provide a set of corrected fiber tracts by removing the false positive subset from the set of input white matter fiber tracts. This may be beneficial because it may provide for an efficient means of removing false positives from the set of input white matter fiber tracts.

It should be noted that once the set of corrected fiber tracts is provided the set of corrected fiber tracts may be set equal to the set of input white matter fiber tracts and all of the steps performed by the execution of the machine-executable instructions may be repeated again or even multiple times. This may provide for a means of iteratively removing the false positive white matter fiber tracts.

In another embodiment the set of corrected fiber tracts is provided in response to a signal from a user interface. For example, the set of input fiber tracts may be rendered on a graphical user interface. The graphical user interface may have a control which enables the sending of a signal which indicates that the false positive subset or a portion of the false positive subset should be removed from the set of input fiber tracts.

In another embodiment execution of the machine-executable instructions further causes the processor to receive a label from the discriminator neural network in response to inputting the set of corrected white matter fiber tracts. Execution of the machine-executable instructions further cause the processor to generate the optimized feature vector using the set of corrected white matter fiber tracts and the generator neural network if the label from the corrected white matter fiber tracts indicates anatomically incorrect. Execution of the machine-executable instructions further cause the processor to receive the set of generated white matter fiber tracts from the generator neural network in response to reinputting the optimized feature vector for the set of corrected white matter fiber tracts. Execution of the machine-executable instructions further cause the processor to construct the false positive subset using the generated set of white matter fiber tracts and the set of input white matter fiber tracts. This embodiment may be beneficial because after the false positive subset has been removed once the entire process can be repeated iteratively.

In another embodiment the false positive subset of the set of input white matter fiber tracts is constructed by comparing each of the set of input white matter fiber tracts to the set of generated white matter fiber tracts. In this embodiment the generated white matter fiber tracts represents the set of white matter fiber tracts which best represent the training data. By for example searching for an optimized feature vector which generates a generator white matter fiber tract that is as close to the input set of white matter fiber tracts this enables the identification of false positives.

In another embodiment each of the set of input white matter fiber tracts has end points and a path. A path as used herein may represent a set of sequentially connected points. Each of the set of generated white matter fiber tracts has an end point and a path. Comparing each of the set of input white matter fiber tracts to the set of generated white matter fiber tracts comprises any one of the following: comparing the end points of the set of white matter fiber tracts to the end points of the set of generated white matter fiber tracts, comparing the set of input white matter fiber tracts to the path of the set of generated white matter fiber tracts, and combinations thereof. In looking at the white matter fiber tracts the path and the starting and end points may be important in determining if they match or not. Having an exact match may however be impossible or not even desirable. In this case different criteria can be used to make the comparison.

In another embodiment comparing the end points of the set of input white matter fiber tracts to the end points of the set of generated white matter fiber tracts comprises generating predetermined end point volumes surrounding end points of the set of generated white matter fiber tracts. In testing is if each of the set of input white matter fiber tracts are both within the predetermined end point volumes of the one of the set of generated white matter fiber tracts. The predetermined end point volumes may for example be volumes which are defined by a predetermined distance to the end points. The comparison may in this example be deemed to be successful if the white matter fiber tract that is being compared has an end point in both the end point volumes.

In another embodiment comparing the end points of the set of input white matter fiber tracts to the end points of the set of generated white matter fiber tracts comprises calculating end point distance between end points of the one of the set of generated white matter fiber tracts and the each of the set of input white matter fiber tracts and then inputting the end point distance into an end point measure function to at least partially provide the comparison. For example, the end point measure function may be a mathematical measure or some other function which is used to make a comparison between the distances. For example, at least squares measure or other measure may be used to compensate if one end point is very close to the reference end point and the other is much further away.

In another embodiment comparing the path of the set of input white matter fiber tracts to the path of the set of generated white matter fiber tracts comprises generating predetermined path volumes surrounding paths of one of the set of generated white matter fiber tracts and then to test if the path of each of the set of input white matter fiber tracts are within the predetermined path volume of the one of the set of generated white matter fiber tracts. For example, the predetermined path volume can be a volume or cylindrical path which surrounds a particular generated white matter fiber tract. If a white matter fiber tract selected from the input white matter fiber tracts is completely within the volume then it may be deemed to have an identical or equivalent path.

In another embodiment comparing the path of the set of input white matter fiber tracts to the path of the set of generated white matter fiber tracts comprises calculating input path distances between the path of one of the set of generated white matter fiber tracts and the path of the set of input white matter fiber tracts and then inputting these distances into a path distance measure function to at least partially provide the comparison. For example a distance can be computed periodically and this can be put into a function such as a least squared function to measure the degree of how well the two paths match each other. A threshold can be set to allow a discrimination between paths that match and those that do not match.

In another embodiment execution of the machine-executable instructions further causes the processor to construct the set of input white matter fiber tracts using a diffusion tensor image of a region of interest descriptive of at least part of a brain according to a fiber tractography algorithm. This fiber tractography algorithm may for example be an automated program that uses a particular algorithm or maybe even uses a neural network to find seed locations. This embodiment maybe beneficial because the removal of the set of false positives may provide a means of providing improvement to a generic fiber tractography algorithm.

In another embodiment execution of the machine-executable instructions further cause the processor to reconstruct the diffusion tensor image for the region of interest using diffusion weighted magnetic resonance imaging data.

In another embodiment the medical imaging system further comprises a magnetic resonance imaging system configured for acquiring magnetic resonance imaging data from an imaging zone. The memory further comprises pulse sequence commands configured for controlling the magnetic resonance imaging system to acquire the diffusion weighted magnetic resonance imaging data for the region of interest according to a diffusion weighted magnetic resonance imaging protocol. The diffusion weighted magnetic resonance imaging acquires a diffusion in a particular direction. It is understood that the diffusion weighted magnetic resonance imaging protocol acquires the diffusion weighting in a sufficient number of directions in order to construct a diffusion tensor image. Execution of the machine-executable instructions further causes the processor to acquire the diffusion weighted magnetic resonance imaging data by controlling the magnetic resonance imaging system with the pulse sequence commands.

In another embodiment the optimized feature vector is calculated by using a search algorithm to iteratively modify elements of the optimized feature vector. This may be performed such that the closest match between the set of generated white matter fiber tracts and the set of input white matter fiber tracts is found.

In another embodiment the optimized feature vector is calculated by back propagation through the generator white matter network. This may be performed such that the back propagation is performed using the set of input white matter fiber tracts. This may be useful because it may find the optimized feature vector which produces a set of generated white matter fiber tracts that is similar to the set of input white matter fiber tracts.

In another aspect the invention provides for a method of operating a medical imaging system using a generative adversarial neural network. The generative adversarial neural network comprises a generator neural network and a discriminator neural network. The generator neural network is configured for outputting a set of generated white matter fiber tracts in response to inputting a feature vector. The discriminator neural network is configured for outputting a label in response to inputting a set of input white matter fiber tracts. The label indicates the set of input white matter fiber tracts as anatomically correct or anatomically incorrect.

The method comprises receiving the set of input white matter fiber tracts. The method further comprises receiving the label from the discriminator neural network in response to inputting the set of input white matter fiber tracts. The method further comprises generating an optimized feature vector using the set of input white matter fiber tracts and the generator neural network if the label indicates anatomically incorrect. The method further comprises receiving the set of generated white matter fiber tracts from the generator neural network in response to inputting the optimized feature vector. The method further comprises constructing a false positive subset of the set of input white matter fiber tracts using the generated set of white matter fiber tracts. The advantages of this have been previously discussed.

In another aspect the invention provides for a computer program product comprising machine-executable instructions for execution by a processor controlling a medical imaging system. The computer program product comprises a generative adversarial neural network. The generative adversarial neural network comprises a generator neural network and a discriminator neural network. The generator neural network is configured for outputting a set of generated white matter fiber tracts in response to inputting a feature vector. The discriminator neural network is configured for outputting a label in response to inputting a set of input white matter fiber tracts. The label indicates the set of input white matter fiber tracts as anatomically correct or anatomically incorrect.

Execution of the machine-executable instructions further causes the processor to receive the set of input white matter fiber tracts. Execution of the machine-executable instructions further causes the processor to receive the label from the discriminator neural network in response to inputting the set of input white matter fiber tracts. Execution of the machine-executable instructions further cause the processor to generate an optimized feature vector using the set of input white matter fiber tracts and the generator neural network if the label indicates anatomically incorrect. Execution of the machine-executable instructions further causes the processor to receive the set of generated white matter fiber tracts from the generator neural network in response to inputting the optimized feature vector.

Execution of the machine-executable instructions further causes the processor to construct a false positive subset of the set of input white matter fiber tracts using the generated set of white matter fiber tracts. The advantages of this have been previously discussed.

It is understood that one or more of the aforementioned embodiments of the invention may be combined as long as the combined embodiments are not mutually exclusive.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) maybe utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection maybe made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks. A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance (MR) data is defined herein as being the recorded measurements of radio frequency signals emitted by atomic spins using the antenna of a Magnetic resonance apparatus during a magnetic resonance imaging scan. A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical imaging system;
Fig. 2 shows a flow chart which illustrates a method of operating the medical imaging system of Fig. 1;
Fig. 3 shows a flow chart which illustrates a further method of operating the medical imaging system of Fig. 1;
Fig. 4 illustrates a further example of a medical imaging system;
Fig. 5 shows a flow chart which illustrates a method of operating the medical imaging system of Fig. 4;
Fig. 6 illustrates a method of comparing white matter fiber tracts; and
Fig. 7 illustrates a further method of comparing white matter fiber tracts.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

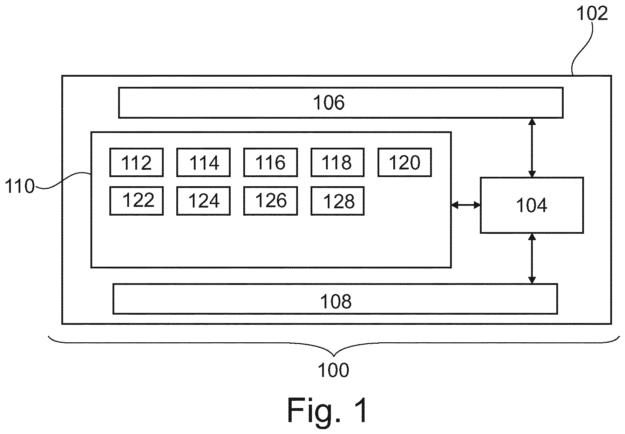

Fig. 1 illustrates an example of a medical imaging system 100. In this example the medical imaging system comprises a computer 102. The computer 102 may be one or more networked computers. The computer 102 comprises a processor 104. The processor 104 is intended to be representative and may represent one or more computing cores as well as one or more processors 104 that are distributed within different computer systems. The processor 104 is shown as being connected to a hardware interface 106 that may enable the processor 104 to communicate with other computer systems as well as control various components of the medical imaging system 100. The processor 104 is further shown as being connected to a user interface 108 which may be a display and/or graphical user interface. The processor 104 is also shown as being in communication with a memory 110. The memory 110 may be any type of memory from which the processor 104 can access. The memory 110 may be any combination of memory which is accessible to the processor 104. This may include such things as main memory, cached memory, and also non-volatile memory such as flash RAM, hard drives, or other storage devices. In some examples the memory 110 may be considered to be a non-transitory computer-readable medium.

The memory 110 is shown as containing a set of machine-executable instructions 112 which enable the processor 104 to control other components of the medical imaging system 100 as well as to perform various data processing and analysis tasks. The memory 110 is further shown as containing a generator neural network 114 and a discriminator neural network 116. The generator neural network 114 and the discriminator neural network 116 form a generative adversarial neural network that may have been trained together. The memory 110 is further shown as containing a set of input white matter fiber tracts 118 that may have been received via data analysis or from a different medical imaging or computer system.

The memory 110 is further shown as comprising a label 120 that was received in response to inputting the set of input white matter fiber tracts into the discriminator neural network 116. The discriminator neural network is configured or trained for outputting a label in response to inputting the set of input white matter fiber tracts. The label indicates whether the set of input white matter fiber tracts are anatomically correct or anatomically incorrect. Anatomically incorrect may be interpreted as comprising false positive white matter fiber tracts.

The memory 110 is further shown as containing an optimized feature vector 122. The optimized feature vector 122 maybe constructed by finding a feature vector that when input into the generator neural network 114 produces a set of generated white matter tracts 124 that closely match the set of input white matter tracts 118. For example, an efficient way of doing this would be to use back propagation and start with the set of generated white matter tracts 124 holding the various nodes and factors within the generator neural network 114 constant and then calculating using the back propagation the optimized feature vector 122.

The memory 110 is further shown as containing a false positive subset 126. The false positive subset is a subset of the set of input white matter tracts 118 that has been identified as being likely false positives. This may be accomplished by for example comparing the set of generated white matter tracts 124 to the set of input white matter tracts 118. Tracts which do not match a starting and ending location as well as a path of the set of generated white matter tracts may be identified as being false positives. The memory 110 is shown as further containing a set of corrected white matter tracts 128 that was formed by removing the false positive subset 126 from the set of input white matter tracts 118.

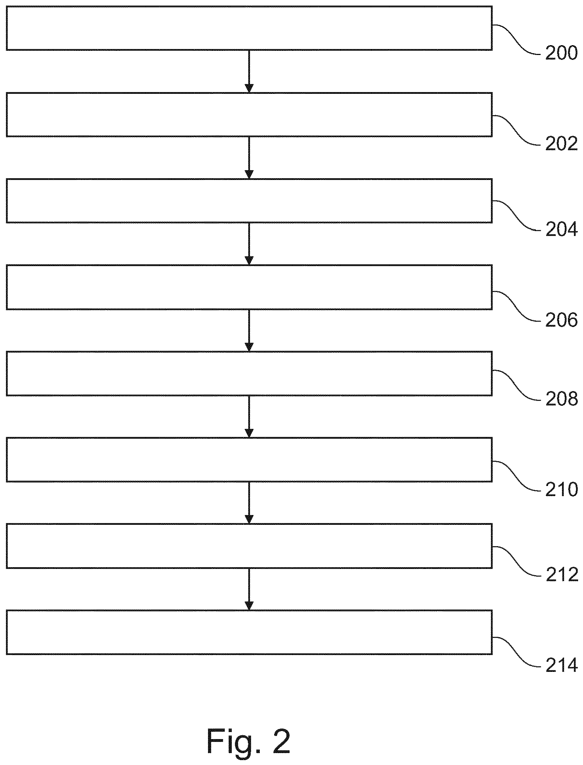

Fig. 2 shows a flowchart which illustrates a method of operating the medical imaging system 100 of Fig. 1. First in step 200 the set of input white matter tracts 118 are received. Next in step 202 the label 120 is received from the discriminator neural network 116 in response to inputting the set of input white matter fiber tracts 118. Then in step 204 the optimized feature vector 122 is generated using the set of input white matter fiber tracts 118 and the generator neural network 114 if the label indicates anatomically incorrect. This for example may be performed by performing back propagation and using the input white matter tracts 118 as the output.

Next in step 206 the set of generated white matter tracts 124 is received from the generator neural network 114 in response to inputting the optimized feature vector 122. Then in step 208 a false positive subset 126 of the set of input white matter tracts 118 is constructed using the generated set of white matter fiber tracts 124 and the set of input white matter tracts 118. Then in step 210 the set of input fiber tracts are optionally rendered on a display. The user interface 108 may for example comprise a display. In step 212 the false positive subset 126 is indicated on the display and/or an anatomically correct subset is indicated on the display. This may be performed by using the false positive subset 126. For example, the false positive subset may be given a different color or highlighting than other fiber tracts. Finally, in step 214, the set of corrected white matter fiber tracts 128 may be provided by removing the false positive subset 126 from the set of input white matter tracts 118.

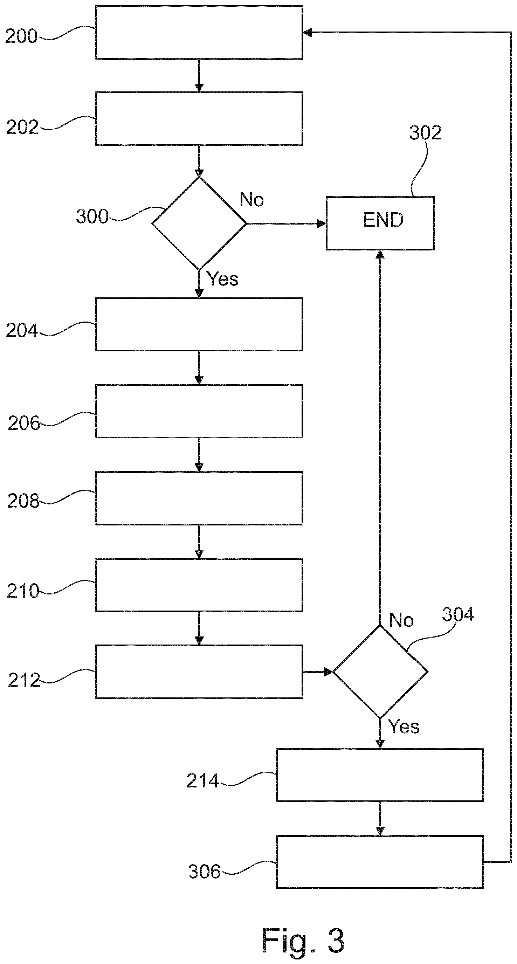

Fig. 3 shows another flowchart which illustrates an alternative method of operating the medical imaging system 100 of Fig. 1. The method in Fig. 3 is similar to the method illustrated in Fig. 2 with the addition of several additional steps. In Fig. 3 the method starts with steps 200 and 202 as was illustrated in Fig. 2. After step 202 the method then proceeds to step 300 which is a question box. In this box the question is 'is the label indicating anatomically incorrect'. If the answer is no then the method proceeds to step 302 which is to render on the display the set of input fiber tracts and the method ends.

When the method reaches step 302 there is no need to proceed further because the set of input white matter fiber tracts do not have the anatomically incorrect label, which is to say the discriminator neural network did not identify any tracts as being abnormal or false positive. If the answer to the question in box 300 is yes, then the method proceeds to steps 204-212 as is illustrated in Fig. 2. The method then proceeds to question box 304. The question in box 304 is 'should the false positive step be removed'. If the answer is no then the method proceeds to step 302 again and the method ends. If the answer is yes then the method proceeds to step 214 and the set of corrected fiber tracts is provided by removing the false positive subset from the set of input white matter tracts.

Then the method proceeds to step 306. In step 306 the set of corrected fiber tracts is then set to the set of input white matter tracts and the method then proceeds back to step 200. The method if therefore iterative. The method proceeds until either the discriminator neural network recognizes all of the white matter fiber tracts as being anatomically correct or until in step 304 a decision is made not to remove any more false positive white matter fiber tracts.

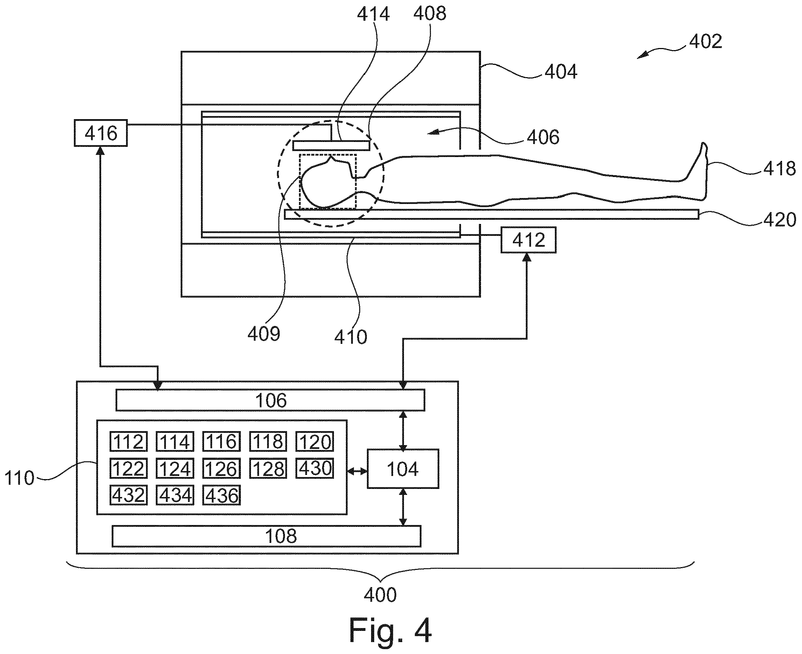

Fig. 4 illustrates a further example of a medical imaging system 400. In this example the medical imaging system further comprises a magnetic resonance imaging system 402. The magnetic resonance imaging system 402 comprises a magnet 404. The magnet 404 is a superconducting cylindrical type magnet with a bore 406 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 406 of the cylindrical magnet 404 there is an imaging zone 108 where the magnetic field is strong and uniform enough to perform magnetic resonance imaging. A region of interest 409 is shown within the imaging zone 408. The magnetic resonance data that is acquired typically acquried for the region of interest. A subject 418 is shown as being supported by a subject support 420 such that at least a portion of the subject 418 is within the imaging zone 408 and the region of interest 409.

Within the bore 406 of the magnet there is also a set of magnetic field gradient coils 410 which is used for acquisition of preliminary magnetic resonance data to spatially encode magnetic spins within the imaging zone 408 of the magnet 404. The magnetic field gradient coils 410 connected to a magnetic field gradient coil power supply 412. The magnetic field gradient coils 410 are intended to be representative. Typically magnetic field gradient coils 410 contain three separate sets of coils for spatially encoding in three orthogonal spatial directions. A magnetic field gradient power supply supplies current to the magnetic field gradient coils. The current supplied to the magnetic field gradient coils 410 is controlled as a function of time and may be ramped or pulsed.

Adjacent to the imaging zone 408 is a radio-frequency coil 414 for manipulating the orientations of magnetic spins within the imaging zone 408 and for receiving radio transmissions from spins also within the imaging zone 408. The radio frequency antenna may contain multiple coil elements. The radio frequency antenna may also be referred to as a channel or antenna. The radio-frequency coil 414 is connected to a radio frequency transceiver 416. The radio-frequency coil 414 and radio frequency transceiver 416 may be replaced by separate transmit and receive coils and a separate transmitter and receiver. It is understood that the radio-frequency coil 414 and the radio frequency transceiver 416 are representative. The radio-frequency coil 414 is intended to also represent a dedicated transmit antenna and a dedicated receive antenna. Likewise the transceiver 116 may also represent a separate transmitter and receivers. The radio-frequency coil 414 may also have multiple receive/transmit elements and the radio frequency transceiver 416 may have multiple receive/transmit channels. For example if a parallel imaging technique such as SENSE is performed, the radio-frequency could 414 will have multiple coil elements.

In this example the subject, 118 is positioned such that the subject's head region is within the region of interest 109 for imaging the brain.

The transceiver 416 and the gradient controller 412 are shown as being connected to the hardware interface 106 of the computer system 101.

The memory 110 is further shown as containing pulse sequence commands that are configured to control the magnetic resonance imaging system 402 to acquire diffusion weighted magnetic resonance imaging data for the region of interest 409 according to a diffusion weighted magnetic resonance imaging protocol. The memory is further shown as containing diffusion weighted magnetic resonance imaging data 432 that was acquired by controlling the magnetic resonance imaging system 402 with the pulse sequence commands 430. The diffusion weighted magnetic resonance imaging data 432 was acquired at a variety of different directions. The memory 110 further shows a diffusion tensor image 434 that was reconstructed from the diffusion weighted magnetic resonance imaging data 432. The memory 110 is shown as further containing a fiber tractography algorithm 436 that takes the diffusion tensor image 434 as input and then outputs the set of input white matter tracts 118.

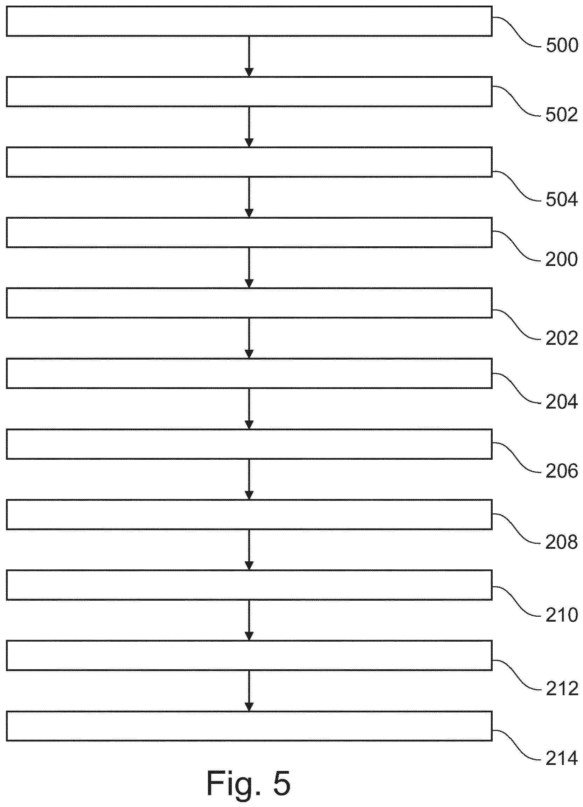

Fig. 5 shows a flowchart which illustrates a method of operating the medical imaging system 400 of Fig. 4. The method in Fig. 5 is similar to the method illustrated in Fig. 2. The method starts with step 500 which is to acquire the diffusion weighted magnetic resonance imaging data 432 by controlling the magnetic resonance imaging system 402 with the pulse sequence commands 430. Next in step 502, the diffusion tensor image 434 is reconstructed from the diffusion weighted magnetic resonance imaging data 432. Next in step 504 the set of input white matter fiber tracts 118 is constructed from the diffusion tensor image 434 using the fiber tractography algorithm 434.

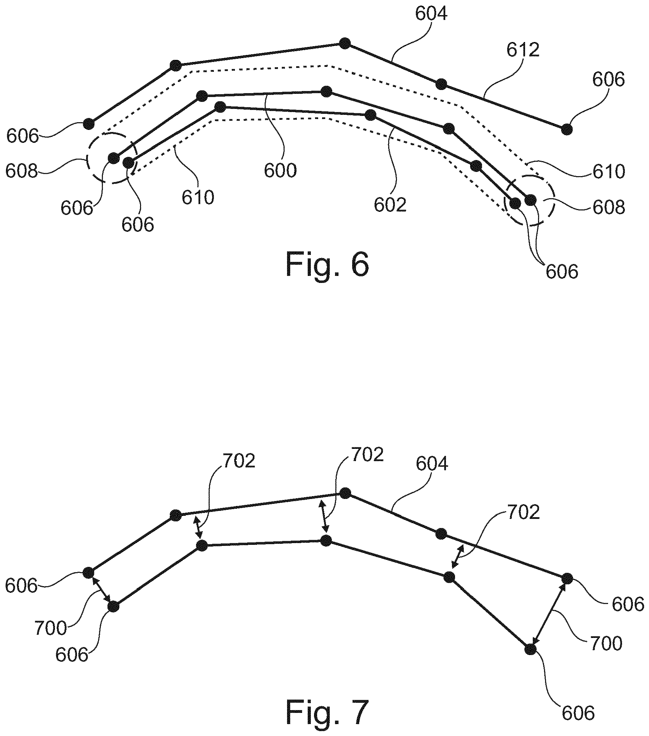

Figs. 6 and 7 illustrate two alternative ways of determining if an input white matter fiber tract is a false positive or not. First in Fig. 6 there is a generated white matter fiber tract 600. Also shown are two input white matter fiber tracts 602 and 604. Each of the white matter fiber tracts has an end point 606. Around the generated white matter fiber tract 600 are predetermined end point volumes 608 which are drawn about the end points 606. There is also a predetermined path volume 610 drawn about the path of the generated white matter fiber tract 600. It can be seen that the end points of the input white matter fiber tract 602 are both within the two different predetermined end point volumes 608. The path of the input white matter fiber tract 602 is also completely within the predetermined path volume 610. The input white matter fiber tract 602 is then determined to be in agreement with the generated white matter fiber tract 600. The other input white matter fiber tract 604 is however completely outside of the predetermined end point volume 608 and the predetermined path volume 610. The input white matter fiber tract 604 is therefore a false positive 612 and would be assigned to the false positive subset 126.

Fig. 7 shows an alternative to the scheme in Fig. 6. In this example the fiber tracts 600 and 604 are illustrated again. Instead of drawing the volumes 608 and 610 it is illustrated that there is an end point distance 700 between the end points 606. A number of path distances 702 are also indicated. They measure the distance between the path of the generated white matter fiber tract 600 and the input white matter fiber tract 604 periodically. The end point distance 700 and the various path distances 702 can be put into a mathematical measure to provide a measure of how closely the input white matter fiber tract 604 matches the generated white matter fiber tract 600. The value of this measure can then be discriminated to determine if the input white matter fiber tract 604 is a false positive or not.

Neuronal fiber tract networks (sets of white matter fiber tracts) in the brain can reveal important clinical information for studying neurological diseases via non-invasive diffusion magnetic resonance imaging (dMRI) technology. Fiber tractography is an algorithmic process for reconstructing structural fiber tracts from dMRI, in vivo. However, the anatomical accuracy of fiber tractography is difficult to measure quantitatively without histological ground truths. In particular, studies using phantom or ex vivo data have shown a prevalence for false positive fiber tracts, which can negatively impact clinical studies used to compare detailed anatomies of disease populations in search of biomarkers. This invention is a deep learning-based framework to detect, visualize, and remove false positive fiber tracts from tractography networks to provide confidence in anatomical accuracy of fiber tractography results for later use in clinical studies.

Neuronal fiber tract networks in the brain can reveal important clinical information for studying neurological diseases via non-invasive diffusion magnetic resonance imaging (dMRI) technology. Fiber tractography is an algorithmic process for reconstructing structural fiber tracts from dMRI, in vivo.

The anatomical accuracy of fiber tractography is difficult to measure quantitatively without histological data of the true brain anatomy. Studies using phantom or ex vivo data have shown a prevalence for false positive fiber tracts, which are fiber tracts that are reconstructed but which are not anatomically correct, ie connections that should not exist. (False negatives are alternatively defined as fiber tracts that exist in the brain for which the tractography algorithm did not reconstruct. These are more difficult to determine because a lack of a connection could be the reason for a particular disease and therefore the algorithm is correct to not reconstruct it.) False positive fiber tracts can negatively impact clinical studies that are used to compare detailed anatomies of disease populations, creating false scientific findings. Current tractography algorithms are unable to effectively detect and remove false positives because there is no ground truth to correctly determine if it is a false positive.

Examples may provide a deep learning-based framework to detect, visualize, and remove false positive fiber tracts from tractography networks to provide confidence in anatomical accuracy of fiber tractography results for later use in clinical studies. There are three main elements of this invention.

The first element is a generative adversarial network (GAN) which is used to learn a distribution of normal anatomical network of fiber tracts for healthy subjects. Within the GAN, a generator network learns a representation for normal fiber tract networks and is used to generate synthetic fiber tract networks from this representation. In parallel a discriminator network is used to classify real and synthetically generated data as "real" (anatomically correct) or "fake" (anatomically incorrect), which updates the accuracy of the generator's representation and synthetic generation. Then, given a reconstructed fiber tract network from a new subject, the discriminator can determine if it is "real" or "fake", ie anatomically correct or incorrect.

The second element is an explanation system to detect and visualize which fibers in the network are responsible for the "fake" classification, ie which fibers are false positives. This is done using a comparison algorithm which navigates the latent representation space (learned by the generator) to generate a similar or close network of fiber tracts with the "real" label. Then by subtraction we can display the differences between the two networks, revealing the set of false positive fiber tracts.

Finally, the third element is a system to remove false positive fiber tracts and provide confidence for the decision. Given a threshold of the difference between the two fiber tract networks, we can remove the false positive fiber tracts and provide additional confidence, by testing this new fiber tract network against the discriminator to classify again if it is "real" or "fake". If the classification is "fake" again, a different removal threshold is used to remove more or fiber tracts or, the process of finding a similar synthetic fiber tract network in step 2 is repeated.

Some examples are constructed by firstly building or training the GAN. While GANs have been widely used for image data, GANs have not been attempted for fiber tract data. Fiber tract data is represented as a set of 3D coordinates, listed sequentially for each fiber tract. The GAN will be trained on this unique data type to learn a latent representation for the fiber tract network and an accompanying discriminator.

Then, given a new subject, the input for the GAN will be a fiber tract network represented by a sequential list of 3D coordinates for each tract and the output of the trained GAN will be a binary class label of "real" or "fake" for the entire dataset. If the new subject fiber tract network is classified as "fake", a synthetic fiber tract network with label "real" will be generated by the GAN that is close to the input subject. Then, given a user input threshold, the difference between these networks will reveal fiber tracts that are responsible for the "fake" label prediction, ie false positives.

Once a set of false positive fiber tracts are located and visualized for a set of thresholds, we can remove these coordinates from the fiber tract network (set of input white matter fiber tracts). We will then re-classify this network using the GAN as "real" or "fake". If it is classified as real, we are done. If it is classified again as "fake", we can either augment the difference threshold and repeat, or generate a new synthetic fiber tract network that is close to this edited fiber tract network (with false positives removed), and repeat.

An additional application of examples is a process of generating phantom fiber tract datasets with ground truths that can be used for research purposes.

Examples may provide for accurate pre-processing of fiber tract networks for clinical research and diagnostic studies related to neurological and psychiatric diseases and disorders.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical imaging system
- 102: computer
- 104: processor
- 106: hardware interface
- 108: user interface
- 110: memory
- 112: machine executable instructions
- 114: generator neural network
- 116: discriminator neural network
- 118: set of input white matter tracts
- 120: label
- 122: optimized feature vector
- 124: set of generated white matter tracts
- 126: false positive subset
- 128: set of corrected white matter tracts
- 200: receive the set of input white matter fiber tracts
- 202: receive the label from the discriminator neural network in response to inputting the set of input white matter fiber tracts
- 204: generate an optimized feature vector using the set of input white matter fiber tracts and the generator neural network if the label indicates anatomically incorrect
- 206: receive the set of generated white matter fiber tracts from the generator neural network in response to inputting the optimized feature vector
- 208: construct a false positive subset of the set of input white matter fiber tracts using the generated set of white matter fiber tracts
- 210: render the set of input fiber tracts on a display
- 212: indicate the false positive subset on the display and/or indicate an anatomically correct subset on the display using the false positive subset
- 214: provide a set of corrected fiber tracts by removing the false positive subset from the set of input white matter fiber tracts
- 300: Is the label anatomically incorrect?
- 302: end
- 304: Remove set of false positive subset from the set of input white matter fiber tracts?
- 306: Set the set of input white matter fiber tracts equal to the set of corrected white matter fiber tracts
- 400: medical imaging system
- 402: magnetic resonance imaging system
- 404: magnet
- 406: bore of magnet
- 408: imaging zone
- 409: region of interest
- 410: magnetic field gradient coils
- 412: magnetic field gradient coil power supply
- 414: radio-frequency coil
- 416: transceiver
- 418: subject
- 420: subject support
- 430: pulse sequence commands
- 432: diffusion weighted magnetic resonance imaging data
- 434: diffusion tensor image
- 436: fiber tractography algorithm
- 500: acquire the diffusion weighted magnetic resonance imaging data by controlling the magnetic resonance imaging system with the pulse sequence commands
- 502: reconstruct the diffusion tensor image for the region of interest using diffusion weighted magnetic resonance imaging data
- 504: construct the set of input white matter fiber tracts using a diffusion tensor image of a region of interest descriptive of at least part of a brain according to a fiber tractography algorithm
- 600: generated white matter fiber tract
- 602: input wite matter fiber tract
- 604: input white matter fiber tract
- 606: endpoint
- 608: predetermined endpoint volume
- 610: predetermined path volume
- 612: false positive
- 700: endpoint distance
- 702: path distance

## Claims

1. A medical imaging system (100, 400) comprising:
- a memory (110) storing machine executable instructions (112) and a generative adversarial neural network (114, 116), wherein the generative adversarial neural network comprises a generator neural network (114) and a discriminator neural network (116), wherein the generator neural network is configured for outputting a set of generated white matter fiber tracts (124) in response to inputting a feature vector, wherein the discriminator neural network is configured for outputting a label (120) in response to inputting a set of input white matter fiber tracts (118), wherein the label indicates the set of input white matter fiber tracts as anatomically correct or anatomically incorrect;
- a processor (104) for controlling the medical imaging system, wherein execution of the machine executable instructions further causes the processor to:
- receive (200) the set of input white matter fiber tracts;
- receive (202) the label from the discriminator neural network in response to inputting the set of input white matter fiber tracts;
- generate (204) an optimized feature vector (122) using the set of input white matter fiber tracts and the generator neural network if the label indicates anatomically incorrect;
- receive (206) the set of generated white matter fiber tracts from the generator neural network in response to inputting the optimized feature vector; and
- construct (208) a false positive subset (126) of the set of input white matter fiber tracts using the generated set of white matter fiber tracts.

2. The medical imaging system of claim 1, wherein execution of the machine executable instructions further causes the processor to:
- render (210) the set of input fiber tracts on a display (108); and
- indicate (212) the false positive subset on the display and/or indicate an anatomically correct subset on the display using the false positive subset.

3. The medical imaging system of claim 1 or 2, wherein execution of the machine executable instructions further causes the processor to provide (214) a set of corrected fiber tracts (128) by removing the false positive subset from the set of input white matter fiber tracts.

4. The medical imaging system claim 3, wherein the set of corrected fiber tracts is provided in response to receiving a signal from a user interface.

5. The medical imaging system of claim 3 or 4, wherein execution of the machine executable instructions further causes the processor to:
- receive the label from the discriminator neural network in response to inputting the set of corrected white matter fiber tracts;
- generate the optimized feature vector using the set of corrected white matter fiber tracts and the generator neural network if the label for the corrected white matter fiber tracts indicates anatomically incorrect;
- receive the set of generated white matter fiber tracts from the generator neural network in response to reinputting the optimized feature vector for the set of corrected white matter fiber tracts; and
- divide the set of white matter fiber tracts into a false positive subset and an anatomically correct subset using the generated set of white matter fiber tracts the set of corrected white matter fiber tracts.

6. The medical imaging system of any one of the preceding claims, wherein the false positive subset of the set of input white matter fiber tracts is constructed by comparing each of the set of input white matter fiber tracts to the set of generated white matter fiber tracts.

7. The medical imaging system of claim 6, wherein each of the set of input white matter fiber tracts has end points (606) and a path (602, 604), wherein each of the set of generated white matter fiber tracts has end points (606) and a path (600), wherein comparing each of the set of input white matter fiber tracts to the set of generated white matter fiber tracts comprises any one of the following:
- comparing the end points of the set of input white matter fiber tracts to the end points of the set of generated white matter fiber tracts; and
- comparing the path of the set of input white matter fiber tracts to the path of the set of generated white matter fiber tracts; and
- combinations thereof.

8. The medical imaging system of claim 7, wherein comparing the end points of the set of input white matter fiber tracts to the end points of the set of generated white matter fiber tracts comprises any one of the following:
- generate predetermined endpoint volumes (608) surrounding endpoints of one of the set of generated white matter fiber tracts, and test if endpoints of the each of the set of input white matter fiber tracts are both within the predetermined endpoint volumes of the one of the set of generated white matter fiber tracts; and
- calculate an endpoint distance (700) between endpoints of the one of the set of generated white matter fiber tracts and the each of the set of input white matter fiber tracts, input the endpoint distance into an endpoint measure function to at least partially provide the comparison.

9. The medical imaging system of claim 7 or 8, wherein comparing the path of the set of input white matter fiber tracts to the path of the set of generated white matter fiber tracts comprises any one of the following:
- generate predetermined path volume (610) surrounding paths of one of the set of generated white matter fiber tracts, and test if the path of the each of the set of input white matter fiber tracts are within the predetermined path volume of the one of the set of generated white matter fiber tracts; and
- calculate input path distances (702) between the path of the one of the set of generated white matter fiber tracts and the path of the set of input white matter fiber tracts, input the input path distances into a path distance measure function to at least partially provide the comparison.

10. The medical imaging system of any one of the preceding claims, wherein execution of the machine executable instructions further causes the processor to construct (504) the set of input white matter fiber tracts using a diffusion tensor image (434) of a region of interest descriptive of at least part of a brain according to a fiber tractography algorithm.

11. The medical imaging system of claim 10, wherein execution of the machine executable instructions further causes the processor to reconstruct (502) the diffusion tensor image for the region of interest using diffusion weighted magnetic resonance imaging data (432).

12. The medical imaging system of claim 11, wherein the medical imaging system further comprises a magnetic resonance imaging system configured for acquiring the diffusion weighted magnetic resonance imaging data from an imaging zone (408), wherein the memory further comprises pulse sequence commands (430) configured to control the magnetic resonance imaging system to acquire the diffusion weighted magnetic resonance imaging data for the region of interest according to a diffusion weighted magnetic resonance imaging protocol, wherein execution of the machine executable instructions further cause the processor to acquire (500) the diffusion weighted magnetic resonance imaging data by controlling the magnetic resonance imaging system with the pulse sequence commands.

13. The medical imaging system of claim 12, wherein the optimized feature vector is calculated using any one of the following:
- using a search algorithm to iteratively modify elements of the optimized feature vector; and
- backpropagation through the generator white matter network.

14. A method of operating a medical imaging system (100, 400) using a generative adversarial neural network (114, 116), wherein the generative adversarial neural network comprises a generator neural network (114) and a discriminator neural network (116), wherein the generator neural network is configured for outputting a set of generated white matter fiber tracts (124) in response to inputting a feature vector, wherein the discriminator neural network is configured for outputting a label (120) in response to inputting a set of input white matter fiber tracts, wherein the label indicates the set of input white matter fiber tracts as anatomically correct or anatomically incorrect, wherein the method comprises:
- receiving (200) the set of input white matter fiber tracts;
- receiving (202) the label from the discriminator neural network in response to inputting the set of input white matter fiber tracts;
- generating (204) an optimized feature vector (122) using the set of input white matter fiber tracts and the generator neural network if the label indicates anatomically incorrect;
- receiving (206) the set of generated white matter fiber tracts from the generator neural network in response to inputting the optimized feature vector; and
- constructing (208) a false positive subset (126) of the set of input white matter fiber tracts using the generated set of white matter fiber tracts.

15. A computer program product comprising machine executable instructions (112) for execution by a processor (104) controlling a medical imaging system (100, 400), wherein the computer program product further comprises a generative adversarial neural network (114, 116), wherein the generative adversarial neural network comprises a generator neural network (114) and a discriminator neural network (116), wherein the generator neural network is configured for outputting a set of generated white matter fiber tracts (124) in response to inputting a feature vector, wherein the discriminator neural network is configured for outputting a label (120) in response to inputting a set of input white matter fiber tracts, wherein the label indicates the set of input white matter fiber tracts as anatomically correct or anatomically incorrect, wherein execution of the machine executable instructions further causes the processor to:
- receive (200) the set of input white matter fiber tracts;
- receive (202) the label from the discriminator neural network in response to inputting the set of input white matter fiber tracts;
- generate (204) an optimized feature vector (122) using the set of input white matter fiber tracts and the generator neural network if the label indicates anatomically incorrect;
- receive (206) the set of generated white matter fiber tracts from the generator neural network in response to inputting the optimized feature vector; and
- construct (208) a false positive subset (126) of the set of input white matter fiber tracts using the generated set of white matter fiber tracts.
